Europäisches Patentamt

European Patent Office

Office européen des brevets

(19)

(11) **EP 1 686 185 A1**

(12) **EUROPEAN PATENT APPLICATION**
published in accordance with Art. 158(3) EPC

(43) Date of publication:
**02.08.2006 Bulletin 2006/31**

(51) Int Cl.:
***C12P 21/00*** *(2006.01)*

(21) Application number: **04799635.0**

(22) Date of filing: **05.11.2004**

(86) International application number:
**PCT/JP2004/016783**

(87) International publication number:
**WO 2005/045053 (19.05.2005 Gazette 2005/20)**

(84) Designated Contracting States:
**AT BE BG CH CY CZ DE DK EE ES FI FR GB GR
HU IE IS IT LI LU MC NL PL PT RO SE SI SK TR**

(30) Priority: **07.11.2003 JP 2003378327**

(71) Applicant: **Ajinomoto Co., Inc.
Tokyo 104-8315 (JP)**

(72) Inventors:
• **UEHARA, Akinori,
c/o Ajinomoto Co., Inc.
Kawasaki-shi,
Kanagawa 210-8681 (JP)**

• **TORIDE, Yasuhiko,
c/o Ajinomoto Co., Inc.
Kawasaki-shi,
Kanagawa 210-8681 (JP)**

(74) Representative: **Strehl Schübel-Hopf & Partner
Maximilianstrasse 54
80538 München (DE)**

(54) **PROCESS FOR PRODUCING LACTIC ACID BACERIUM CULTURE CONTAINING BACTERIOCIN AND METHOD OF STORING FOOD USING THE SAME**

(57)    Lactic acid bacterium culture containing protease-resistant bacteriocin can be produced by cultivaiting lactic acid bacteria such as a genus Weissella and so on and the shelf stability of a food can be improved by using the said culture in the food.

EP 1 686 185 A1

**Description**

Technical Field

The present invention relates to 1) a method for producing lactic acid bacterium culture containing bacteriosin, 2) a method for preserving food products using the lactic acid bacterium culture containing bacteriocin, and 3) a screening method for a lactic acid bacterium producing bacteriocin.

Background Art

For the purpose of preventing the decay and quality deterioration of food products, food preservatives have been added to various food products. As such food preservatives, chemically synthesized food preservatives have mainly been used so far. However, chemically synthesized food preservatives are sometimes concerned in the light of safety issues such as persistence or toxicity. So as to solve such problems, it is expected to develop a safe antimicrobial substance derived from traditional foods.

Meanwhile, lactic acid bacteria are useful microorganisms having been used traditionally in production of various fermented food products (including fermented beverages) such as soy sauce, soybean paste (miso), pickles and Japanese sake. Additionally, lactic acid bacteria have been used in the process of producing fermented food products. This is ascribed to the inhibition of the growth of contaminated bacteria in the production process and the resulting products, due to the pH reduction of the systems with lactic acid produced via lactic acid fermentation, so that the decay and quality deterioration of such food products can be prevented. Additionally, it is also verified that antimicrobial substances produced by lactic acid bacteria are also useful for the prevention of the decay and quality deterioration of food products.

Among antimicrobial substances, proteinaceous antimicrobial substances produced by various bacteria are referred to as bacteriocin. Among varieties of bacteriocin produced by lactic acid bacteria, nisin is approved as a GRAS substance by FDA and also approved as a safe substance with antimicrobial activity by WHO and FAO. Further, nisin is utilized as a food preservative in 50 countries or more all over the world.

However, disadvantageously, known varieties of bacteriocin including nisin are readily decomposed with proteases. In other words, bacteriocin is readily decomposed with proteases produced by Aspergillus oryzae and so on in the process of producing fermented food products like sake, soy sauce and soybean paste (miso). Therefore, the bacteriocin cannot maintain satisfactory antimicrobial activities. In the case of nisin addition prior to pasteurization for sake (Publication No.JP06-319516) and in the case of the addition of acidocin 8912 as another bacteriocin variety (Publication No.JP06-319516), for example, it is reported that antimicrobial effects cannot be obtained because these varieties of bacteriocin are decomposed with enzymes such as protease in unprocessed sake. Currently, no report exists indicating that lactic acid bacteria produce protease-resistant bacteriocin.

Additionally, processed meat products such as ham and sausage are spontaneously fermented due to microorganisms inherently existing in the raw materials thereof or microorganisms contaminated during the production process, so that preferable flavor and preservability can be imparted to. For the purpose of stabilization the product quality, shortening of production time period and prevention of the growth of hazardous microorganisms, currently, the starter culture method has been developed (Science and Technology of Lactic Acid Bacteria, Association Press Center, p.239, 1996).

For example, Chung et al. disclose the effect of immersing uncooked meat in nisin solution or the effect of nisin on fresh edible meat preliminarily inoculated with certain bacterial species. According to the report, nisin used in fresh edible meat loses its activity in a very short time (Env. Microbiol. 55: (6) p. 1329-1333 (1989)). This is due to the nisin decomposition with protease such as cathepsin in edible meat causing the loss of the antimicrobial activity in a very short time.

So as to prevent such nisin decomposition with enzymes as described above, an invention is reported, which includes heat-treating edible meat and subsequently applying lanthionine-base bacteriocin such as nisin to the surface of the heat-treated edible meat (Publication No.JP06-22685). However, edible meat should be heat-treated before nisin addition. Therefore, the range of the use of the invention is more or less limited.

Currently in food industries, thus, providing a protease-resistant bacteriocin applicable to a wide range of food products including fermented food products and processed meat products is strongly desired.

Disclosure of the Invention

As described above, lactic acid bacteria have been used traditionally for the treatment of various food products including fermented food products and have never caused any safety concerns. Additionally, antimicrobial substances produced by lactic acid bacteria are considered to be safer than chemically synthesized substances. Thus, the objectives of the invention are to provide 1) a method for producing lactic acid bacterium culture containing protease-resistant bacteriocin; 2) a method for preserving food products using the culture containing the said bacteriocin; and 3) a screening method for a lactic acid bacterium producing protease-resistant bacteriocin.

In order to solve the problems, the inventors isolated lactic acid bacteria existing in fermented food products such as fermented milk, and screen bacterial strains which produce novel protease-resistant bacteriocin. Consequently, the inventors successfully isolated a lactic acid bacterium producing the substance. The inventors confirmed that the bacteriocin produced by the bacterial strain is a novel substance. And, the invention is described as follows.

(1) A method for producing a lactic acid bacterium culture containing bacteriocin which is resistant to proteases.

(2) The method described in (1), wherein the lactic acid bacterium belongs to any one of a genus Weissella, Pediococcus, Lactobacillus or Leuconostoc.

(3) The method described in (2), wherein the lactic acid bacterium belonging to a genus Weissella is any one of Weissella sp. FERM P-19577,Weissella cibaria JCM12495, Wissella confusa JCM1093, Weissella hellenica JCM10103,Weissella kandleri JCM5817, Weissella minor JCM1168, Weissella paramesenteroides JCM9890 or Weissella thailandensis JCM10694.

(4) The method described in (2), wherein the lactic acid bacterium belonging to a genus Pediococcus is Pediococcus pentosaceus.

(5) The method described in (2), wherein the lactic acid bacterium belonging to a genus Lactobacillus is any one of Lactobacillus plantarum, Lactobacillus salivarius or Lactobacillus pentosus.

(6) The method described in (2), wherein the lactic acid bacterium belonging to a genus Leuconostoc is any one of Leuconostoc citreum, Leuconostoc pseudomesenteroides, Leuconostoc argentinum, Leuconostoccarnosum or Leuconostoc mesenteroides.

(7) A method for preserving a food product, wherein the lactic acid bacterium culture described in any one of (1) to (6) is used in a process of producing the food product.

(8) The method described in (7), wherein the food product is a fermented food product or a processed meat product.

(9) A method for screening a lactic acid bacterium which produces bacteriocin comprising a step of screening lactic acid bacterium culture with an antimicrobial activity even in the presence of a protease.

The invention is now described in detail hereinbelow.

In Armenia known also as a country where people enj oy longer longevity, traditionally, a great number of healthy foodproducts to be formulated for sickness have been known. For example, the food products include lactic acid food products such as Matsoon and Narine, dry apricot, red wine, jyesiin and tiinaff. With attention mainly focused on fermented milk Matsoon eaten in Armenia and koji being a raw material for fermented food products, the inventor developed research works.

In this invention, "bacteriocin which is resistant to proteases" or "protease-resistant bacteriocin" means the bacteriocin which has an antimicrobial activity even in the presense of a protease such as a protease derived fromAspergillus oryzae. Bacteriocin whose antimicrobial activity is lowered by amylases is also included.

The lactic acid bacterium culture containing "bacteriocin which is resistant to proteases" or "protease-resistant bacteriocin" means the culture which forms an inhibitory zone of the indicator strain in the following method more specifically:

(1) A lactic acid bacterium culture is prepared according to an ordinary cultivation method (or a cultivation method for separating microorganisms). The pH of the lactic acid bacterium culture is adjusted to pH5.5 to 6.0 with sodiumhydroxide solution. Subsequently, the culture is centrifuged at 12,000 rpm for 10 minutes and filtrated with Disposable Syringe Filter Unit "Dismic-25cs", Cellulose Acetate 0.45μm (ADVANTEC Inc).The filtered liquid is used as a sample. If the antimicrobial activity of the sample is low, the sample needs to be concentrated up to 4 times under reduced pressure at ambient temperature. If necessary, it is concentrated to 10 times.

(2) Listeria innocua ATCC33090T, Bacillus circulans JCM2504T, Bacillus coagulans JCM2257, Micrococcus luteus IFO12708, Bacillus subtilis JCM1465T, Bacillus subtilis IAM1381, Lactococcus lactis sub sp. Lactis ATCC19435, Enterococcus faecium JCM5804T, Enterococcus faecium JCM5803T, Lactobacillus plantarum ATCC14917T and Lactobacillus sakei JCM1157T are used as an indicator strain and the indicator having the highest antimicrobial activity is selected by measuring the antimicrobial activities by the spot-on-lawn method described later or counting the colony forming unit.

(3) A protease derived from Aspergillus (UMAMIZYME G, Amano Enzyme Co) is used as an enzyme.

(4) 10 to 100 unit/ml of the protease described in (3) is added to the sample described in (1) and reacted at 30°C for more than one hour.

(5) The indicator strain exhibiting the highest antimicrobial activity described in (2) is spread on a medium plate such as MRS medium plate where the indicator can grow.0.01 ml of the protease treated sample described in (4) is dropped on the center of the medium plate at the optimal temperature for the growth of the indicator (37°C for Listeria innocua, Bacillus coagulans, Enterococcus faecium or Pediococcus pentosaceus and 30°C for others) for 20 to 24 hours. Then, the inhibitory zone of the indicator is observed.

Lactic acid bacteria which produce bacteriocin resistant to proteases according to this invention are separated from fermented food products and so on. It is needless to say that lactic acid bacteria with antimicrobial activity obtainable by the screening method described below may be used as well. In other words, any lactic acid bacteria producing protease-resistant bacteriocin may be used satisfactorily, with no specific limitation to the source from which the bacteria are separated. As a result of examinations by the inventors, the inventors discovered that among lactic acid bacteria, genera Weissella, Pediococcus, Lactobacillus,Leuconostoc ,and so on produce the intended protease-resistant bacteriocin. It is needless to say that any lactic acid bacteria other than those described above can be used as long as the lactic acid bacteria produce protease-resistant bacteriocin.

By using the lactic acid bacterium culture containing protease-resistant bacteriocin obtained by cultivating these lactic acid bacteria producing the protease-resistant bacteriocin in a process of producing various types of fermented food products such as soy sauce, miso and fish sauce and various types of processed meat products such as ham and sausage, the decay and quality deterioration of the intended food products can be prevented. The bacteriocin may be isolated and used. Otherwise, the culture containing bacteriocin may be used as it is, with no isolation of the bacteriocin. Because purification procedures such as isolation are generally laborious, preferably, the lactic acid bacterium culture itself is added in a process of producing various types of fermented food products. Further, the culture containing the protease-resistant bacteriocin may satisfactorily be added in one portion or plural portions, in a process of producing fermented food products, processed meat products and so on. Satisfactorily, how many portions the bacteriocin or the broth is divided into for addition may be determined freely.

So as to obtain the intended lactic acid bacterium culture containing protease-resistant bacteriocin, the lactic acid bacteria should be cultivated. Cultivation conditions such as cultivation temperature, culture time, cultivation method and medium may be the ordinary condition used in cultivating lactic acid bacteria. Additionally, routine separation and purification methods such as gel filtration may be used satisfactorily in case of isolation. The lactic acid bacterium culture or the lactic acid bacteria culture in this invention means a medium containing cultivated lactic acid bacteria or a medium which cultivated lactic acid bacteria is removed from by centrifuge or the like. And, the medium may be liquid, solid or gel-like. In case that a liquid medium is used, it is sometimes described as a lactic acid bacterium broth. It is needless to say that a lactic acid bacterium culture includes a lactic acid bacterium broth. In addition, a dried powder of liquid lactic acid bacterium/bacteria culture by spray drying, freeze-drying or the like, a concentrated liquid or paste of liquid lactic acid bacterium/bacteria culture by filtration, evaporation or the like, or a fraction with antimicrobial activities of liquid lactic acid bacterium/bacteria culture by gel filtration, chromatography or the like is included in the lactic acid bacterium/ bacteria culture of this invention.

The lactic acid bacterium culture containing protease-resistant bacteriocin may be added to any food products, with no specific limitation. Most preferably, the culture is added to fermented food products and processed meat products where microorganisms are involved in their production process.

Fermented food products include soy sauce, fish sauce, sake, soybean paste miso, pickles, cheese and so on. These are just examples. The lactic acid bacterium culture containing protease-resistant bacteriocin may satisfactorily be used for those other than the examples described above.

Herein, traditionally, sodium chloride has been used as a bacteriostatic agent in fermented food products. Owing to the increase of demands toward low salt diet in recent years and the advantage of expediting the protein decomposition rate by lowering or removing salt in the fermentation, research works have been made to use bacteriocin such as nisin in fermented food products. Because nisin and the existing varieties of bacteriocin are decomposed by proteases existing in the production processes, however, none of the bacteriostatic effect is currently observed. Even for the processes of producing fermented food products, the lactic acid bacterium culture containing bacteriocin with protease resistance can be used.

Additionally, the processed meat products include for example ham and sausage. It is needless to say that these are just example. Therefore, the culture containing the protease-resistant bacteriocin may satisfactorily be used for those other than the examples just described above.

Foodproducts suchas fermented foodproducts and processed meat products produced by the addition of the lactic acid bacterium culture containing protease-resistant bacteriocin have extremely high shelf stability.

The screening method for a lactic acid bacterium producing the protease-resistant bacteriocin as an important aspect of the invention is now described in the following example where such lactic acid bacterium is separated from a fermented food product Matsoon.

A sample collected from fermented milk Matsoon which is one of fermented food products is cultivated in a medium where a lactic acid bacterium can grow, for example the MRS medium (Table 1) or the M17 medium (Table 2) at 30 °C to 37 °C, whrein the amount of the sample to the medium is 0.5 %. The culture time is one day, 5 days and 10 days. After completion of the cultivation, the broth is spread and cultivated on the agar medium (agar at 1.2 %) containing 0.5 % calcium carbonate. From the resulting colonies, lactic acid bacteria are collected.

Table 1. Composition of MRS medium

| Composition of MRS medium (Merck) | |
|---|---|
| Peptone | 10.0 g/l |
| Lab-Lemco's Powder | 8.0 g/l |
| Yeast extract | 4.0 g/l |
| Glucose | 20.0 g/l |
| Tween 80 | 1.0 g/l |
| Dipotassium hydrogen phosphate | 2.0 g/l |
| Sodium acetate | 5.0 g/l |
| Ammonium citrate | 2.0 g/l |
| Magnesium sulfate · 7H$_2$O | 0.20 g/l |

Table 2. Composition of M17 medium

| Composition of M17 medium (Merck) | |
|---|---|
| Soybean meal-derived peptone | 5.0 g/l |
| Meat-derived peptone | 2.5 g/l |
| Casein-derived peptone | 2.5 g/l |
| Yeast extract | 2.5 g/l |
| Meat extract | 5.0 g/l |
| D(+)-Lactose | 5.0 g/l |
| Ascorbic acid | 0.5 g/l |
| β-Glycerophosphate sodium | 19.0 g/l |
| Magnesium sulfate | 0.25 g/l |

The collected lactic acid bacteria are cultivated in the heretofore described manner. Then, these lactic acid bacteria are inoculated and cultivated for 24 hours on a plate of the MRS agar medium to which filtrated Umamizyme G (Amano Enzyme co)being a protease derived from Aspergillus oryzaeproteases were added. Subsequently, the Lactobacilli AOAC medium (Table 3) into which an indicator strain is preliminarily mixed is overlaid on the plate for cultivation for 24 hours, to form an inhibitory zone of the indicator strain.

Table 3. Composition of Lactobacilli AOAC medium

| Composition of Lactobacilli AOAC medium (Difco) | |
|---|---|
| Peptonized milk | 15.0 g/l |
| Yeast extract | 5.0 g/l |
| Dextrose | 10.0 g/l |
| Tomato juice | 5.00 g/l |
| Monopotassium dihydrogen phosphate | 2.0 g/l |
| Polysorbate 80 | 1.0 g/l |

As the method for adding the protease, methods other than the method to mix the protease into the agar medium may be used, such as the following methods.

1) A method to mix the protease together with an indicator strain into the medium.

2) A method to spread the protease on the agar medium.

3) A method to add the protease when the colonies of lactic acid bacteria are cultivated. In this case, the protease may be added at the start of cultivation, during cultivation or on the completion of cultivation.

4) A method to observe the formation of the inhibitory zone by adding onto a plate where an indicator strain is mixed an appropriate amount of the sample where the protease was added after cultivating colonies of lactic acid bacteria and then disinfecting or killing the bacteria in the broth. It is described again that the method is not limited to the methods 1) to 4). Additionally, the protease is not limited to Umamizyme G.

Then, an evaluation is done by antimicrobial spectral analysis. Using the spot-on-lawn method to spot the supernatant of the lactic acid bacterium culture with antimicrobial activities on a plate for examining the antimicrobial activity described below, the antimicrobial spectrum is examined.

First, a sample with antimicrobial activity is prepared. The culture liquid of the bacterial strain having an antimicrobial activity obtained by the aforementioned method is centrifuged at 10, 000 rpm for 10 minutes to obtain a culture supernatant. And then the supernatant is filtrated through a filter to obtain an aseptic sample.' The sample is diluted by every 2 fold to prepare a dilution series to $2^{11}$ dilutions. In case that the activity is low, the sample is concentrated by every 2 fold to prepare a concentration series to $2^{-3}$ dilutions under reduced pressure at ambient temperature.

Then, the indicator strain to be mixed on the plate for examining the antimicrobial activity is cultivated. The indicator strain in Table 4 are cultivated in the TSBYE medium (Tables 5 and 6) or the MRS medium. Bacteria of the genera Bacillus and Micrococcus are cultivated by using a shaker but the other bacteria are stationarily cultivated. Additionally, Bacillus coagulans, Listeria, Pediococcus and Enterococcus are cultivated at 37 °C, while the other are cultivated at 30 °C.

Table 4. Indicator strain for evaluating Antimicrobial activity

| Name of bacterial strain | Medium/Temperature (°C) | Cultivation method |
|---|---|---|
| Bacillus coagulans JCM2257 | TSBYE/37 | Shaker culture |
| Bacillus subtilis JCM1465T | TSBYE/30 | Shaker culture |
| Bacillus subtilis IAM1381 | TSBYE/30 | Shaker culture |
| Bacillus circulans JCM2504T | TSBYE/30 | Shaker culture |
| Micrococcus luteus IFO12708 | TSBYE/30 | Shaker culture |
| Listeria innocua ATCC33090T | TSBYE/37 | Stationary culture |
| Pediococcus pentosaceus JCM5885 | MRS/37 | Stationary culture |
| Enterococcus faecalis JCM5803T | MRS/37 | Stationary culture |
| Enterococcus faecium JCM5804T | MRS/37 | Stationary culture |
| Lactococcus lactis subsp. lactis ATCC19435 | MRS/30 | Stationary culture |
| Lactobacillus plantarum ATCC14917T | MRS/30 | Stationary culture |
| Lactobacillus sakei subsp. sakei JCM1157T | MRS/30 | Stationary culture |
| Leuconostoc mesenteroides subsp. mesenteroides JCM6124T | MRS/30 | Stationary culture |
| Lactobacillus kimchii JCM10707T | MRS/30 | Stationary culture |

Table 5. Composition of TSBYE medium

| Composition of TSBYE medium | |
|---|---|
| TSB medium | 30.0 g/l |
| Yeast extract (Difco) | 6.0 g/l |

Table 6. Composition of TSB medium

| Composition of Bacto tryptic soy broth (TSB) medium (Difco) | |
|---|---|
| Pancreatic digest of casein | 17.0 g/l |
| Enzymatic digest of soybean meal | 3.0 g/l |
| Dextrose | 2.5 g/l |
| Sodium chloride | 5.0 g/l |
| Dipotassium monohydrogen phosphate | 2.5 g/l |

Further, a plate for examining the antimicrobial activity is prepared. 10 ml of the MRS agar medium (agar at 1.2 %) and 5 ml of the Lactobacilli AOAC agar medium (agar at 1.2 %) are separately sterilized at 121 °C for 15 minutes and are then kept warm at 55 °C. The sterilized MRS agar medium is poured into an aseptic petri dish and is then left to stand in a clean bench for one hour. Subsequently, 50 μl of a broth of an indicator strain is mixed to the Lactobacilli AOAC agar medium kept warm at 55 °C. The broth is overlaid on the MRS plate. The lid of the plate is opened in the clean bench (for about 15 minutes), to dry the surface.

10-μl each of the prepared sample with the antimicrobial activity as prepared above is dropped to the plate. Then, the lid is closed to leave as it is for about one hour, to dry the plate. The plate is incubated at a suitable temperature of each indicator strain for 20 hours, to examine the formation of an inhibitory zone. Herein, the antimicrobial activity (AU/ml) is defined as follows.

```
Antimicrobial activity (AU/ml) = (maximum dilution ratio for forming the inhibitory circle) × 1000/10
```

The samples of which the antimicrobial spectrum was analyzed in such manner had protease resistance and showed a wide range of antimicrobial spectrum.

The bacteriological profile of the lactic acid bacterial strain AJ110263 selected by the method described above was examined. Based on the homology analysis in terms of the nucleotide sequence of 16S ribosome DNA (rDNA) (Altschul, S. F., Madden T.F., Schaffer, A. A., Zhang, J., Zhang, Z., Miller, W., and Lipman, D. J. (1997) Gapped BLAST and PSI-BLAST: a new generation of protein database search programs. Nucleic Acids Res. 25: 3389-3402.), the bacterial strain had 98.22 % homology to Weissella confusa strain ATCC 10881 (Table 7). For the homology assessment, herein, the type culture which is deposited at ATCC was used.

Table 7. 16S rDNA homology of strain AJ110263

| 16S rDNA homology | Weissella confusa | 98.22 % |
|---|---|---|
| | Weissella viridescens | 95.20 % |
| | Weissella minor | 92.54 % |
| | Weissella kandleri | 92.01 % |
| | Weissella halotolerans | 87.39 % |
| | Weissella paramesenteroide | 86.25 % |
| | Lactobacillus mali | 78.17 % |
| | Pediococcus parvulus | 77.58 % |

It was considered that the basic profile (Table 8) of the bacterial strain coincided with the general properties of lactic acid bacteria and that the sugar fermentation pattern (Table 9) was similar to that of Weissella confusa. However, the bacterial strain showed a different fermentation pattern for L-arabinose and did not have 100 % homology on the basis of 16S rDNA, so that it was found that the bacterial strain is a novel bacterial strain different from any known bacteria. Thus, the bacterial strain was defined as Weissella sp. AJ110263. The bacterial strain was deposited at the International Patent Organism Depositary (IPOD), the National Institute of Advanced Industrial Science and Technology (AIST). Its accession number is FERM P-19577.

Table 8. Basic profile of Lactic acid bacterium strain AJ110263

| Cell morphology | Short bacillus (0.8 - 1.0 $\times$ 1.0 - 1.5 $\mu$m) |
| --- | --- |
| Gram staining | (+) |
| Spore | (-) |
| Mobility | (-) |
| Colony morphology (medium: MRS medium) (cultivation temperature:30 °C) (culture time: 24 hr) | circle overall periphery smooth lowly protruded shape gloss opaque |
| Cultivation temperature (37 °C/45 °C) | (+/-) |
| Catalase | (-) |
| Acid/gas generation (glucose) | (+/-) |
| O/F test (glucose) | (+/+) |
| Growth at pH 9.6 | (+) |
| Growth in NaOH (6.5 %) | (+) |

Table 9. Fermentation profile of sugars of Lactic acid bacterium strain AJ110263

| Fermentation profile of sugars | | |
| --- | --- | --- |
| Fermentation (+) | L-arabinose | arbutin |
| | D-xylose | esculin |
| | Galactose | salicin |
| | Glucose | cellobiose |
| | Fructose | maltose |
| | Mannose | purified sugar |
| | N-acetylglucosamine | gentiobiose |
| | Amygdalin | gluconate |

(continued)

| Fermentation profile of sugars | | |
|---|---|---|
| Fermentation (-) | Glycerol | trehalose |
| | Erythritol | inulin |
| | D-arabinose | melezitose |
| | Ribose | raffinose |
| | L-xylose | starch |
| | Adonitol | glycogen |
| | β-methyl-D-xylose | xylitol |
| | Sorbose | D-tulanose |
| | Rhamnose | D-lyxose |
| | Dulcitol | D-tagatose |
| | Inositol | D-fucose |
| | Mannitol | L-fucose |
| | Sorbitol | D-arabitol |
| | α-methyl-D-mannose | L-arabitol |
| | α-methyl-D-glucose | 2-ketogluconic acid |
| | Lactose | 5-ketogluconic acid |
| | Melibiose | |

Best Mode of Carrying Out the Invention

The invention is now described below with reference to the following examples. However, the examples never limit the invention.

Example 1

Weissella sp. AJ110263 (FERM P-19577) separated from fermented milk Matsoon and Pediococcus pentosaceus JCM5885, Pediococcus pentosaceus JCM5890, Lactobacillus plantarum JCM1149 and Lactobacillus salivarius JCM1231 obtained from the type cultures were preliminarily cultivated and then cultivated in the MRS medium (Table 1). The Weissella sp. was cultivated at 30 °C, while the other bacterial strains were cultivated at 37 °C. The lactic acid bacteria were inoculated on the plate of the MRS medium where 0 U/ml (not added), 200 U/ml and 400 U/ml of Umamizyme G shown in Table 3 were added, and cultivated for 24-hour.

Herein, the cultivation was conducted by charging 100 ml of the MRS medium in a 500-ml Sakaguchi's flask and then inoculating 100 μl of each of the preliminary broth for cultivation at a shaker of 100 strokes /min.

Subsequently, the Lactobacilli AOAC medium where Lactobacillus sakei strain JCM1157 was mixed as an indicator strain, was overlaid. These plates were incubated for 24 hours. Consequently, inhibitory zones of the indicator strain were formed (Table 10). These results indicated that each strain produces the protease-resistant bacteriocin.

Table 10. Diameter (mm) of Inhibitory Zone of PRB producing strain

| Strain | Protease (U/ml) | | |
|---|---|---|---|
| | 0 | 200 | 400 |
| Weissella sp. AJ110263 | 7 | 10 | 10 |
| Pediococcus pentosaceus JCM5885 | 15 | 15 | 15 |
| Pediococcus pentosaceus JCM5890 | 10 | 12 | 12 |
| Lactobacillus plantarum JCM1149 | 15 | 17 | 23 |

(continued)

| Strain | Protease (U/ml) | | |
|---|---|---|---|
| | 0 | 200 | 400 |
| Lactobacillus salivarius JCM1231 | 13 | 18 | 18 |

Lactobacillus sakei strain JCM1157 was used as the indicator strain. Numeric values in the table express the diameter of the growth inhibitory zone.

Example 2

Lactococcus lactis NCDO497 (a nisin A producer) and Lactococcus lactis NCIMB702054 (a nisin Z producer) were cultivated in the MRS medium at 30 °C. In the same manner as in Example 1, the antimicrobial activity was evaluated, using Lactobacillus sakei strain JCM1157 as an indicator strain.

Additionally, the antimicrobial activity was evaluated, by spotting 10 μl of a 1000 IU/ml solution of Nisin A, ICN Biomedical Inc. instead of using the nisin producer, on the plate of the MRS agar medium.

In the absence of protease, an inhibitory zone of the indicator strain was formed. In the presence of protease, the activity was lowered in case the protease concentration was higher (Table 11).

Table 11. Diameter of Inhibitory Zone of Strain not producing PRB

| Strain | Protease (U/ml) | | |
|---|---|---|---|
| | 0 | 200 | 400 |
| Nisin A added (no use of any bacterial strain) | 30 | ND | ND |
| Lactococcus lactis NCDO497 (a nisin A producer) | 30 | 13 | ND |
| Lactococcus lactis NCIMB702054 (a nisin Z producer) | 30 | 13 | ND |
| Lactobacillus sakei strain JCM1157 was used as the indicator strain. Numeric values in the table express the diameter of the inhibitory zone. ND = not detected | | | |

Example 3

The strains Weissella sp. AJ110263 (FERM P-19577), Pediococcus pentosaceus JCM5885, Lactococcus lactis NCDO497 (a nisin A producer) and Lactobacillus sakei JCM 1157 were cultivated. The broth was centrifuged at 10,000 rpm for 10 minutes, to obtain culture supernatants. After adding 2000 U/ml of Umamizyme to the supernatants and treating by the enzyme for 24-hour, the supernatants were filtrated with a filter (DISMIC25CS, ADVANTEC; 0.45 μm), to prepare aseptic samples. Using the spot-on-lawn method, the antimicrobial spectra were examined. Consequently, Weissella sp. AJ110263 (FERM P-19577) and Pediococcus pentosaceus JCM5885 kept their antimicrobial activities even after the protease treatment, compared with the broth of the nisin-producing bacterium and Lactobacillus sakei JCM1157 which does not produce bacteriocin (Table 12). This indicated that Weissella sp. AJ110263 (FERM P-19577) and Pediococcus pentosaceus JCM5885 produce the protease-resistant bacteriocin.

Table 12

| Indicator \\ Sample | Weissella sp. AJ110263 | Pediococcus pentosaceus JCM5885 | Lactococcus lactis NCDO497 | Lactobacillus sakei JCM1157T |
|---|---|---|---|---|
| Listeria innocua ATCC33090T | 50 | 50 | ND | ND |
| Bacillus circulans JCM2504T | 100 | 100 | 50 | 50 |
| Bacillus coagulans JCM2257 | 50 | 100 | ND | ND |
| Micrococcus luteus IFO12708 | 100 | 100 | ND | ND |
| Bacillus subtilis JCM1465T | 100 | 100 | ND | 50 |
| Lactococcus lactis subsp. lactis ATCC19435 | 50 | 50 | ND | ND |
| Enterococcus faecium JCM5804T | 50 | 100 | ND | ND |
| Enterococcus faecalis JCM5803T | 100 | 100 | ND | 50 |
| Lactobacillus plantarum ATCC14917T | 100 | 100 | ND | 50 |
| Lactobacillus sakei JCM1157T | 50 | 50 | ND | ND |

After the broth was treated with protease, the supernatants were evaluated by the spot-on-lawn method. Numeric values express antimicrobial activity. Antimicrobial activity (AU/ml) = maximum dilution ratio for forming inhibitory circle $\times$ 1000/10; ND = not detected.

Example 4

Culture supernatants of strains Weissella sp. AJ110263 (FERM P-19577), Pediococcus pentosaceus JCM5885, Lactobacillus plantarum JCM1149, Lactobacillus salivarius JCM1231, Leuconostoc citreum JCM9698, Leuconostoc pseudomesenteroides JCM9696, JCM11045 and Lactococcus lactis NCIMB702054 (a bacterium producing nisin Z) were treated with the enzyme as described in Example 3. Bacillus subtilis IAM1381 was used as an indicator strain. As the enzyme, Umamizyme G derived from Aspergillus oryzae was used as described in Example 3. in addition, α-amylase derived from Bacillus subtilis (Wako Pure Chemical Ltd) was added to the lactic acid bacterium culture broth in an amount of 100U/ml and submitted to the reaction at 30°C for more than one hour. And then, the antimicrobial activity was evaluated by the spot-lawn method wherein Bacillus subtilis IAM1381 was used as an indicator strain in the same manner, to study the effect of α-amylase to the antimicrobial activity. As shown in Table 13, Weisella sp.AJ110263 (FERM P-19577), Weissella cibaria JCM12495, Weissella confusa JCM1093, Weissella hellenica JCM10103, Weissella kandleri JCM5817, Weissella minor JCM1168, Weissella paramesenteroides JCM9890, Weissella thailandensis JCM10694, Pediococcus pentosaceus JCM5885, Lactobacillus plantarum JCM1149, Lactobacillus salivarius JCM1231, Lactobacillus pentosus JCM1558, Leuconostoc citreum JCM9698, Leuconostoc pseudomesenteroides JCM9696, JCM11045, Leuconostoc argentinum JCM11052, Leuconostoc carnosum JCM9695 and Leuconostoc mesenteroides JCM6124 kept their antimicrobial activities even after the protease treatment. Consequently, it was found these strains produced protease resistant bacteriocin.

Table 13.Residual Antimicrobial Activity after Enzyme Treatment

| | Residual antibiotic activity | | |
| --- | --- | --- | --- |
| | control | umamizyme | $\alpha$-amylase |
| **Nisin producer** | | | |
| **Lactococcuslactis NCIMB702064** | 100 | nd | 100 |
| **Protease-resisitant-bacteriocin (PRB) producer** | | | |
| **Weissella sp. AJ110263** | 100 | 100 | 30 |
| **Weissella cibaria JCM12495** | 100 | 100 | 30 |
| **Weissella confusa JCM1093** | 100 | 70 | 50 |
| **Weissella hellenica JCM10103** | 100 | 100 | 40 |
| **Weissella kanrileri JCM5817** | 100 | 100 | 40 |
| **Weissella minor JCM1168** | 100 | 100 | 40 |
| **Weissella paramesenteroides JCM9890** | 100 | 100 | 70 |
| **Weissella thailandensis JCM10694** | 100 | 100 | 40 |
| **Pediococcus pentosaceus JCM5885** | 100 | 90 | 30 |
| **Lactobacillus plantarum JCM1149** | 100 | 80 | 30 |
| **Lactobacillus salivarius JCM1231** | 100 | 80 | 30 |
| **Lactobacillus pentosus IAM1558** | 100 | 100 | 30 |
| **Leuconostoc citreum JCM9698** | 100 | 80 | 40 |
| **Leuconostoc pseudomesenteroides JCM9696** | 100 | 100 | 50 |
| **Leuconostoc pseudomesenteroides JCM11045** | 100 | 100 | 50 |
| **Leuconostoc argentinum JCM11052** | 100 | 100 | nd |
| **Leuconostoc carnosum JCM9695** | 100 | 100 | 30 |
| **Leuconostoc mesenteroides JCM6124** | 100 | 100 | 40 |

Example 5

Soybean (10 g) and pure water (10 ml) were individually put into six Erlenmeyer flasks (200-ml volume) and sterilized in an autoclave at 120 °C for 30 minutes. After cooling, 0.04 g of koj i mold (Purple 1, NO.1 bacterium for soy sauce) was added, and cultivated statically at 30 °C for 2 days. 40 ml of sterile pure water were added to cultivated sample (Sample No.1), 40 ml of salt solution which adjusted the salt content of the sample to 18 % to Sample No.2, 40 ml of a culture supernatant of Lactococcus lactis NCIMB702054 (the bacterium producing nisin Z) to Sample No.3 and 40 ml of a culture supernatant of Weissella sp. AJ110263 (FERM P-19577)to Sample No.4, 40 ml of a culture supernatant of Pediococcus pentosaseceus JCM5885 to Sample 5 and 40 ml of a culture supernatant of Lactobacillus salivarius JCM1231 to Sample 6. The resulting mixtures were then adjusted to pH 6.5 to 7.0, using 6N hydrochloric acid and 6N NaOH passed through a filter.

200 μl of Bacillus subtilis IAM1381 cultivated in the TSBYE medium by a shaker at 30°C for 20 hours was additionally inoculated, mixed thoroughly and cultivated at 30 °C. On days 1, 2 and 7 of the cultivation, the broth was collected to count the viable cells of Bacillus subtilis IAM1381 on the GAM agar medium (GAM bouillon "NISSUI" , Nissui Pharmaceutical Co., Ltd.). In the pure water sample, the contaminating bacterium Bacillus subtilis IAM1381 existed at $10^8$ cells per gram or more. In the 18% salt sample, no contamination occurred. In case of the addition of the nisin supernatant with no salt, meanwhile, nisin was decomposed with proteases derived from the koji mold on day 1 and thereafter. Thus, $10^8$ cells per gram or more contaminated therein and no antimicrobial effect was observed.

In case of using the supernatant of Weissella sp. AJ110263 (FERM P-19577), Pediococcus pentosaseceus JCM5885 and Lactobacillus salivarius JCM1231, however, the contaminating bacterium Bacillus subtilis IAM1381 was never observed on the first day of the fermentation up to day 7 (Table 14).

Table 14.Test to use bacteriosin as a substitute for salt in the manufacturing process of soy sauce

| No | Salt | Liquid of lactic acid bacterium | Bacteriosin | On day 1 of fermentation | | On day 7 | |
|---|---|---|---|---|---|---|---|
| | | | | Glu (mg/dl) | viable cell count BS | Glu (mg/dl) | viable cell count BS |
| 1 | not added | absence | not added | 400 | 3 × 10^8 | 980 | 3 × 10^7 |
| 2 | 18% | absence | not added | 90 | ND | 490 | ND |
| 3 | not added | Lactococcus Lactis NCIMB702054 | nisin*2 | 430 | 3 × 10^7 | 920 | 3 × 10^7 |
| 4 | not added | Weissella sp. AJ110263 | PRB | 580 | ND | 1,140 | ND |
| 5 | not added | Pediococcus pentosaceus JCM5885 | PRB | 450 | ND | 1,064 | ND |
| 6 | not added | Lactobacillus salivarius JCM1231 | PRB | 460 | ND | 1,176 | ND |
| PRB:Protein Resistant Bacteriosin | | | | | | | |

Example 6

The broths obtained by cultivating Lactococcus lactis NCIMB702054 (a strain producing NisinZ) and Weissella sp.AJ11026 (FERM P-19577) in MRS culture media were adjusted to pH 5.5 with sodium hydroxide. Subsequently, the supernatants were prepared by removing bacteria from the pH adjusted culture liquids by centrifuge. The broths containing lactic acid bacteria and the supernatants were used in the experiment described below.

5 g of ground meat of Black hair Japanese beef were individually put into six aseptic Falcon tubes (Becton Dickinson Co. 50-ml volume) and 5 ml of saline were added to the meat (Sample No.1), 5 ml of 18% salt solution to Sample No.2, 5 ml of the supernatant of Lactococcus lactis NCIMB702054 to Sample No.3, 5 ml of the broth of Lactococcus lactis NCIMB702054 to Sample No.4, 5 ml of the supernatant of Weissella sp. AJ11026 to Sample No.5, 5 ml of the broth of Weissella sp. AJ11026 to Sample No. 6. Further, Listeria innocua ATCC33090 statically cultivated in TSBYE medium at 37°C for 24 hours was added to the samples in the amount of $10^8$ cfu/ml and the samples were aged at ambient temperature. The samples aged for one day and seven days were collected to count the viable cells of Listeria innocua ATCC33090 in the Listeria selection medium (Oxoid Inc.). As shown in Table 15, in the saline sample, the contaminating bacterium Listeia innocua ATCC33090 existed at $10^6$ cfu/ml or more. In the 18% salt solution sample, the contaminating bacterium Listeia innocua ATCC33090 existed at $10^5$ cfu/ml or more. In case of the addition of the culture broth or the supernatant of nisin producing lactic acid bacteria, the contaminating bacterium Listeia innocua ATCC33090 existed at $10^6$ cfu/ml or more since nisin was decomposed with the protease derived from meat (cathepsin) . In case of using the culture broth or the supernatant of Weissella sp. AJ11026 (FERM P-19577), however, the contaminating bacterium Listeia innocua ATCC33090 could be reduced to $10^3$ cfu/ml on the first day of aging up to day 7.

Table 15.

| No. | salt | lactic acid Bacterium | (bacteriocin) | aging FAA ($\mu$mol/g) | day 0 cfu/ml | aging FFA ($\mu$mol/g) | day 1 cfu/ml | aging FFA ($\mu$mol/g) | day 7 cfu/ml |
|---|---|---|---|---|---|---|---|---|---|
| 1 | not added | not added | | 72 | 2*10^6 | 70 | 5*10^7 | 100 | 2*10^6 |
| 2 | 18% | not added | | 66 | 4*10^6 | 73 | 6*10^5 | 70 | 2*10^5 |
| 3 | not added | Lactococcus lactis #120 | nisin Z sup. | 72 | 4*10^6 | 75 | 2*10^7 | 88 | 4*10^6 |
| 4 | not added | Lactococcus lactis #120 | nisin Z broth | 83 | 4*10^6 | 81 | 1*10^6 | 84 | 4*10^6 |
| 5 | not added | Weissella sp AJ10155 | sup. PRB | 82 | 4*10^6 | 93 | 4*10^3 | 111 | 1*10^3 |
| 6 | not added | Weissella sp AJ10156 | broth PRB | 84 | 4*10^6 | 87 | nd | 104 | 2*10^2 |

PRB:Protein Resistant Bacteriosin

FFA: Free Amino Acid

Additionally, the antimicrobial spectra were examined. It was indicated that the bacteriocin had a growth-inhibiting effect over Listeria causing food poisoning and Bacillus subtilis being disadvantageous in the manufacturing process of producing soy sauce andmisopaste, besides Enterococcus faecium.

Stability against pH and temperature was also examined. The novel bacteriocin retained about 50 % of the activity even at pH 2 to 4. In a wide range of pH 2 to pH 11, the antimicrobial activity was stable. Particularly, the bacteriocin had a strong antimicrobial activity around pH 4 to pH 6. Additionally even after heating at 100 °C for 10 minutes, the bacteriocin retained about 50 % of the activity. Thus, it was shown that the bacteriocin had great thermal stability.

Industrial Applicability

A very excellent method for preserving food products can be provided, by adding the lactic acid bacterium culture containing protease-resistant bacteriocin produced by lactic acid bacteria such as Weissella sp., Pediococcus pentosaceus, Lactobacillus plantarum and Lactobacillus salivarius in a manufacturing process of fermented food products, processed meat products ,and so on.

**Claims**

1. A method for producing a lactic acid bacterium culture containing bacteriocin which is resistant to proteases.

2. The method according to claim 1, wherein the lactic acid bacterium belongs to any one of a genus Weissella, Pediococcus, Lactobacillus or Leuconostoc.

3. The method according to claim 2, wherein the lactic acid bacterium belonging to a genus Weissella is any one of Weissella sp. FERM P-19577,Weissella cibaria JCM12495, Wissella confusa JCM1093, Weissella hellenica JCM10103,Weissella kandleri JCM5817, Weissella minor JCM1168, Weissella paramesenteroides JCM9890 or Weissella thailandensis JCM10694.

4. The method according to claim 2, wherein the lactic acid bacterium belonging to a genus Pediococcus is Pediococcus pentosaceus.

5. The method according to claim 2, wherein the lactic acid bacterium belonging to a genus Lactobacillus is any one of Lactobacillus plantarum, Lactobacillus salivarius or Lactobacillus pentosus.

6. The method according to claim 2, wherein the lactic acid bacterium belonging to a genus Leuconostoc is any one of Leuconostoc citreum, Leuconostoc pseudomesenteroides, Leuconostoc argentinum, Leuconostoccarnosum or Leuconostoc mesenteroides.

7. A method for preserving a food product, wherein the lactic acid bacterium culture according to any one of claims 1 to 6 is used in a process of producing the food product.

8. The method according to claim 7, wherein the food product is a fermented food product or a processed meat product.

9. A method for screening a lactic acid bacterium which produces bacteriocin comprising a step of screening lactic acid bacterium culture with an antimicrobial activity even in the presence of a protease.

**INTERNATIONAL SEARCH REPORT**

| International application No. |
|---|
| PCT/JP2004/016783 |

| A. CLASSIFICATION OF SUBJECT MATTER |
|---|
| Int.Cl⁷  C12P21/00, A23L1/31, A23L1/317, A23L3/3526, A23L3/3571, C12Q1/04, C12Q1/37//(C12P21/00, C12R1:225)(C12P21/00, C12R1:01) |

According to International Patent Classification (IPC) or to both national classification and IPC

| B. FIELDS SEARCHED |
|---|
| Minimum documentation searched (classification system followed by classification symbols) |
| Int.Cl⁷  C12P21/00, A23L1/31, A23L1/317, A23L3/3526, A23L3/3571, C12Q1/04, C12Q1/37//(C12P21/00, C12R1:225)(C12P21/00, C12R1:01) |

Documentation searched other than minimum documentation to the extent that such documents are included in the fields searched

Electronic data base consulted during the international search (name of data base and, where practicable, search terms used)
  MEDLINE, BIOSIS/WPI(DIALOG)

| C. DOCUMENTS CONSIDERED TO BE RELEVANT |||
|---|---|---|
| Category* | Citation of document, with indication, where appropriate, of the relevant passages | Relevant to claim No. |
| X | Skytta E. et al., Production and characterization of antibacterial compounds produced by Pediococcus damnosus and Pediococcus pentosaceus, J.Appl.Bacteriol., 1993, Vol.74, pages 134 to 142 | 1-9 |
| X | Lavermicocca P. et al., Purification and characterization of novel antifungal compounds from the sourdough Lactobacillus plantarum strain 21B, Appl.Environ.Microbiol., 2000, Vol.66, pages 4084 to 4090 | 1-9 |
| X | Sato R. et al., Characteristic evaluation of lactic acid bacteria collections based on the bacteriocin productivity, Rep.Natl. Food Res. Inst., 2002, No.66, pages 15 to 20 | 1-9 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 21 December, 2004 (21.12.04) | 11 January, 2005 (11.01.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

16

| | **INTERNATIONAL SEARCH REPORT** | International application No. |
| | | PCT/JP2004/016783 |

C (Continuation).   DOCUMENTS CONSIDERED TO BE RELEVANT

| Category* | Citation of document, with indication, where appropriate, of the relevant passages | Relevant to claim No. |
|---|---|---|
| X | Hara K. et al., Inhibitory activity in starter cultures being used for fermented milk and antimicrobial substance produced by Lactobacillus delbrueckii subsp. lactis 5001, Animal Science and Technology, 1994, Vol.65, pages 674 to 681 | 1-9 |
| X | Jay J.M., Antimicrobial properties of diacetyl, Appl.Environ.Microbiol., 1982, Vol.44, pages 525 to 532 | 1-9 |
| X | JP 8-289770 A  (Asama Chemical Co., Ltd.), 05 November, 1996 (05.11.96), Full text (Family: none) | 1-9 |
| X | JP 2000-300284 A  (Kabushiki Kaisha Seibutsu Kassei Kenkyusho), 31 October, 2000 (31.10.00), Full text (Family: none) | 1-9 |

Form PCT/ISA/210 (continuation of second sheet) (January 2004)